# EUROPEAN PATENT SPECIFICATION

(11) **EP 2 900 119 B1**
(45) Date of publication and mention of the grant of the patent: **11.03.2020**
(21) Application number: 13766532.9
(22) Date of filing: 25.09.2013
(51) Int. Cl.: A61B 1/12, A61B 1/00

(54) **HYDROPHOBIC MEMBRANE FOR A LENS AND METHOD OF PROTECTING A LENS WITH SUCH A MEMBRANE**
HYDROPHOBE MEMBRAN FÜR EINE LINSE UND VERFAHREN ZUM SCHUTZ EINER LINSE MIT SOLCH EINER MEMBRAN
MEMBRANE HYDROPHOBE POUR LENTILLE ET PROCÉDÉ DE PROTECTION DE LENTILLE AU MOYEN DE LADITE MEMBRANE

(30) Priority: 26.09.2012 FR 1259037
(43) Date of publication of application: 05.08.2015
(73) Proprietor: Sofradim Production, 01600 Trévoux (FR)
(72) Inventor: LADET, Sébastien, F-69300 Caluire et Cuire (FR); VESTBERG, Robert, 69260 Charbonnières les Bains (FR)
(74) Representative: Delorme, Nicolas
(86) International application number: PCT/EP2013/069940
(87) International publication number: WO 2014/048972

(56) References cited:
- EP-A1- 1 982 640
- EP-A2- 2 486 845
- WO-A1-2012/101226
- CN-A- 102 354 058
- DE-A1-102010 003 720
- JP-A- 2005 052 229
- US-A- 5 575 756
- US-A1- 2009 269 587
- US-A1- 2011 260 348
- US-A1- 2012 088 066

## Description

The present invention relates to a disposable flexible membrane that is suitable for being adhered to an objective lens, especially a camera objective for laparoscopic operations.

A surgeon performing a laparoscopic operation generally uses a camera for viewing the internal organs. Now, physiological fluids soil the lens of the camera, and as a result the visibility is reduced as the intervention proceeds. It is then necessary to remove the camera in order to be able to clean the lens. The loss of visibility during the operation increases the risk of a surgical error. Furthermore, cleaning of the lens complicates the intervention. The steps for removing and reinserting the camera prolong the duration of the intervention, the costs and the duration of the anaesthesia for the patient.

In order to overcome these drawbacks, cameras exist, the lens of which has a permanent hydrophobic coating so as to make the lens self-cleaning. However, over time, repeated sterilization of the camera before each intervention leads to erosion of the coating, which loses its efficacy. Furthermore, the initial formation of the permanent hydrophobic coating on the lens is very expensive and restoring it entails expensive steps.

The present invention is directed towards overcoming the abovementioned drawbacks. To this end, the invention proposes a disposable flexible membrane according to claim 1.

Documents US 5,575,756 and WO 2012/101226 disclose membranes according to the preamble of claim 1.

By "flexible" is meant in the present application the feature according to which an element, such as a membrane or a film, is able to be bent easily without breaking.

Throughout the document, the term "surface with reversible adhesiveness" means a surface which has the capacity of adhering to another surface of a material such as glass, while at the same time being able to be removed manually without leaving any residue or irreversible degradation on the said other surface. In particular, such a "surface with reversible adhesiveness" is capable of adhering temporarily to the said other surface.

It is understood that, in the document, the term "disposable membrane" is synonymous with a single-use membrane. Specifically, the cost of use of the membrane is low and it can be removed manually from the surface to be protected in order to be discarded after each use.

In the present document, the term "transparent membrane" means a membrane that is substantially transparent to wavelengths of the visible range.

The term "hydrophobic" means a compound which repels or is repelled by water. In particular, in the present document, a hydrophobic compound is a compound which repels or is repelled by biological fluids.

Another aspect of the invention is a method for temporarily protecting the objective lens of a camera comprising the step of manually positioning a membrane as described herein onto said lens and further manually removing said membrane from said lens.

Thus, the membrane according to the invention allows ease of use of a camera for penetrating inside the human body and which comes into contact with biological fluids, over several surgical interventions. The camera, which is intended, for example, for laparoscopic operations, is sterilized and then the new, sterile membrane according to the invention is placed on the objective lens of the camera before the intervention. Because of its flexible nature, the membrane may be easily and manually positioned onto the objective lens. In addition, because of its flexibility, the membrane matches the surface of the lens. The positioning of the membrane onto the objective lens does not necessitate any specific tool. The user may place the membrane onto the lens simply with a simple grasping tool. During the operation, the biological fluids slide over the hydrophobic surface of the first face of the membrane according to the invention, which ensures good visibility throughout the intervention. Specifically, the hydrophobic properties of the membrane according to the invention give the surface of the lens a water-repellent nature. The affinities with any material containing water, such as biological fluids, are reduced, even if the water is present in small amount.

The reversible adhesive nature of the second face of the membrane according to the invention enables the membrane to be removed manually at the end of the intervention without leaving any residues on the lens. Again, because of its flexible nature, the membrane may be easily manipulated and removed from the objective lens, with a simple grasping tool. At each intervention, a new membrane according to the invention is used, erosion of the hydrophobic properties of a permanent coating known in the cameras of the prior art does not take place with the membrane according to the invention. Furthermore, the duration of the intervention is reduced, and the risks of errors are limited. Since the membranes according to the invention are inexpensive, they are less expensive to use than a lens covered with a permanent coating.

In the present patent application, the use of the membrane will be described in relation with the lens of a camera objective intended for laparoscopic operations. However, the membrane according to the invention may be used for any apparatus comprising an objective intended to come into contact with an aqueous medium that is liable to soil the objective lens. It may especially be an objective for submarine photography.

Advantageously, the second face of the membrane according to the invention comprises an adhesive material, such an adhesive material especially affords the necessary adhesiveness of the membrane for maintenance to the lens during the intervention while at the same time enabling manual removal of the membrane after use.

Preferably, the adhesive material is chosen from epoxy adhesives and pressure-sensitive adhesive (PSA) polymers such as vinyl polymers, especially vinyl ethers, ethylene-vinyl acetates (EVA), natural and butyl rubbers, nitriles, acrylic resins, silicone rubbers, especially styrene-butadiene-styrene (SBS) copolymers, styrene block copolymers (SBC), styrene-ethylene/butylene-styrene (SEBS) copolymers, styrene-ethylene/propylene (SEP) copolymers and styrene-isoprene-styrene (SIS) copolymers, and mixtures thereof. These adhesive materials afford a reversible adhesive nature to the membrane according to the invention. They are moreover compatible with use in a sterile surgical medium. The styrene-butadiene-styrene copolymers (also known by the abbreviation SBS) are commercially available, for example, from the company BASF under the trade name Styroflex®.

According to one embodiment, the hydrophobic surface comprises a hydrophobic compound grafted onto the first face or a hydrophobic compound in the form of a deposited layer. Such an embodiment makes it possible especially to ensure good solidarity between the first face of the membrane according to the invention and the hydrophobic compound. Furthermore, since the hydrophobic compounds are formed from polymer materials, they limit the formation of fog on the membrane according to the invention during an intervention, when compared with the surface of a glass lens whose surface is colder. The materials used for depositing a hydrophobic layer are especially naturally hydrophobic polymers such as fluoro polymers, polyolefins and other modified polymers bearing hydrophobic groups such as groups based on fatty acids and fluoro hydrocarbons, and mixtures thereof.

Advantageously, the hydrophobic surface comprises texturing, the dimensions of which range from 1 nanometre to 20 micrometres. The term "texturing" means irregularities of the profile of the surface, the dimensions of which, especially perpendicular to the surface, range from 1 nanometre to 20 micrometres. This texturing allows drops of water placed on the surface to exhibit a contact angle of greater than or equal to 90°. The first face of the membrane according to the invention then has a surface of improved hydrophobic nature, which increases the self-cleaning capacity of the membrane.

According to the invention, the hydrophobic surface comprises particles whose dimensions range from 1 nanometre to 20 micrometres and whose surface is hydrophobic. Texturing of the surface and an improved hydrophobic nature are thus obtained.

For example, the particles comprise cellulose nanofibres also known as NFC (nano-fibrillated cellulose), and, preferably, the product of the reaction of the nano-fibrillated cellulose (NFC) with a hydrophobic fluoro compound such as tridecafluoro(1,1,2,2-tetrahydrooctyl)trichlorosilane so as to form particles whose surface is hydrophobic.

According to one embodiment, the particles comprise a hydrophobic material, which facilitates the production of a surface of improved hydrophobic nature.

According to an alternative embodiment, the texturing is formed by etching the hydrophobic surface, for example via a lithography technique.

According to a particular embodiment, the membrane according to the invention comprises a film, namely a flexible film, comprising a polymer material such as polyvinyl, poly(ethylene terephthalate) (or PET), acrylic, and mixtures thereof, a first surface and an opposite second surface, the flexible film being arranged between the first face and the second face of the membrane. The flexible film gives the membrane mechanical strength and also suppleness and the flexibility necessary for the handling of the membrane and reversible adhesiveness on an objective lens.

According to an embodiment, the flexible film comprises a plasticizer. The presence of a plasticizer in the composition of the flexible film makes it possible especially to improve the flexibility, the resistance and the resilience of the flexible film, which may be easier to manipulate. Examples of plasticizers that may be used are phthalates such as bis (2-ethylhexyl) phthalate, glycols, polyethers and alkyl citrates, and mixtures thereof.

Preferably, the polymer material of the flexible film is an adhesive polymer, such as styrene-butadiene-styrene copolymer, and forms the adhesive material of the second face of the membrane. The membrane according to the invention is thus easier to design and, as a result, is less expensive to manufacture.

According to one embodiment, the polymer material of the flexible film is a hydrophobic material, such as a fluoro polymer, especially polytetrafluoroethylene, fluorinated ethylene-propylene, a fluorinated elastomer, and mixtures thereof, and forms the hydrophobic material of the first face of the membrane.

According to one embodiment, the membrane according to the invention also comprises fixing means that are adapted to be fixed onto a metal substrate. It is thus possible to reinforce the fixing of the membrane onto the lens by attaching the membrane to a metal frame in which the lens is placed.

In particular, the membrane according to the invention may comprise a circular portion intended to be adhered to the lens and the fixing means comprise at least one tab extending radially to the circular portion. For example, this tab may be self-adhesive and intended to be bonded to the metal frame of the camera. However, the tab is not intended to be adhered to the lens.

Other aspects, aims and advantages of the present invention will emerge more clearly on reading the following description of two embodiments thereof, which are given as non-limiting examples and with reference to the attached drawings. All the elements represented in the figures are not necessarily to scale, so as to improve their readability. In the rest of the description, for the sake of simplicity, identical, similar or equivalent components of the various embodiments bear the same numerical references.
Figure 1 is a top view of a membrane according to one embodiment of the invention.
Figure 2 is a section in a direction perpendicular to one face of a membrane according to one embodiment of the invention.
Figure 3 is a section in a direction perpendicular to one face of a membrane according to another embodiment of the invention.

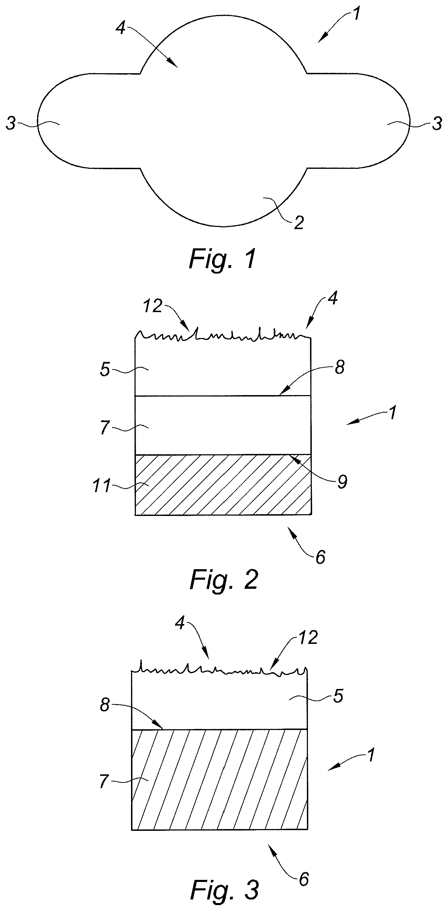

Figure 1 is a top view of a membrane 1 according to the invention, which is flexible, disposable and adapted to be adhered to a circular lens of an objective. The objective may be that of a camera for operations within the human body and intended to come into contact with biological fluids, such as laparoscopic operations.

The membrane 1 has a circular portion 2 intended to be adhered to the lens and fixing means that are in the form of two tabs 3 extending radially to the circular portion 2. These tabs 3 are adapted to be fixed onto a metal support, such as a metal fixing frame of the lens. According to one possibility, the tab 3 may consist of an optionally transparent adhesive strip.

According to a possibility, not shown, in which the lens is not circular, the portion of the membrane 1 according to the invention which is intended to be adhered to the lens is non-circular but has a shape substantially similar to that of the lens.

According to a variant not shown, the fixing means may consist, for example, of a single tab 3 or of more than two tabs 3. Globally, the fixing means may have any form adapted to the fixing of the membrane 1 onto a metal substrate serving as a support for the lens. However, these fixing means are not intended to be fixed onto the lens.

Figure 1 also shows a first face 4 of the membrane 1 comprising a hydrophobic surface such that soiling cannot adhere thereon. The hydrophobic property of the surface may be obtained by grafting a hydrophobic compound onto the first face 4 of the membrane 1. It may also result from the formation of a layer 5 (Figure 2) of a hydrophobic compound via a deposition technique such as plasma-assisted CVD (chemical vapour deposition).

With reference to Figure 2, a second face 6 of the membrane 1, opposite the first face 4, comprises a surface with reversible adhesiveness so as to be able to adhere the circular portion 2 of the membrane 1 to the lens during the use of the objective. Next, at the end of use, the membrane 1 can be manually removed without substantially leaving any residues. The adhesive nature of the surface is thus reversible. The lens may then be reused for a new intervention.

Figure 2 shows the membrane 1 in a direction perpendicular to the first face 4. In this embodiment, the membrane 1 comprises a flexible film 7 arranged between the first face 4 and the second face 6 of the membrane 1. This flexible film 7 comprises a polymer material such as polyvinyl, poly(ethylene terephthalate), acrylic, and mixtures thereof. According to another possibility, the flexible film 7 also comprises a plasticizer. The plasticizer may be chosen especially from phthalates such as bis(2-ethylhexyl)phthalate, glycols, polyethers and alkyl citrates, and mixtures thereof.

A layer 5 of a hydrophobic material placed on a first surface 8 of the flexible film 7 forms the hydrophobic surface of the membrane 1. A layer 11 of an adhesive material covers a second surface 9 of the flexible film 7 so as to give the second face 6 of the membrane 1 reversible adhesiveness.

The layer 5 of hydrophobic material has a textured surface 12. Specifically, the surface of the layer 5 has an irregular profile with variations from 1 nanometre to 20 micrometres. These profile irregularities reinforce the hydrophobic nature of the surface of the first face 4 and give it an improved hydrophobic nature, which improves the self-cleaning properties of the membrane 1. The surface texturing may be obtained, for example, by etching the hydrophobic material, obtained especially by lithography.

According to another possibility, not shown, the first surface 8 of the flexible film 7 is first etched to obtain adequate texturing. A layer 5 of hydrophobic material is then deposited on this first textured surface 8 while reproducing the irregularities of its profile, so as to obtain a textured surface 12.

According to an embodiment variant, the surface of improved hydrophobic nature 12 of the membrane 1 is obtained by particles whose sizes range from 1 nanometre to 20 micrometres and whose surface is hydrophobic. These particles may consist especially of a hydrophobic material. The particles comprise, for example, nanofibrillated cellulose (NFC) which has reacted with a hydrophobic fluoro compound, such as tridecafluoro(1,1,2,2-tetrahydrooctyl)trichlorosilane, making the surface of the nanofibres hydrophobic. In this case, the cellulose nanofibres at the surface which has been made hydrophobic are diluted in ethanol and then sprayed onto the first surface 8 of the film until a layer 5 whose textured surface 12 has an improved hydrophobic nature is obtained.

The second surface 9 of the film covered with the layer 11 of adhesive material has reversible adhesiveness. This reversible adhesive nature is especially obtained with an adhesive material chosen from vinyl polymers, epoxy adhesives, acrylic adhesives such as a pressure-sensitive acrylic resin, and styrene-butadiene-styrene copolymers.

Figure 3 shows a flexible membrane 1 which differs from that shown in Figure 2 in that the flexible film 7 comprises a styrene-butadiene-styrene copolymer forming the adhesive material of the second face 6 of the membrane 1. In this embodiment, it is not necessary to envisage the deposition of a layer 11 of an adhesive material onto the second surface 9 of the flexible film 7, which reduces the cycle time and the manufacturing costs of the membrane 1.

During the use of the flexible membrane 1 on a lens of a laparoscopic camera objective, the camera is first sterilized. Next, the adhesive surface of the second face 6 of the circular portion 2 of the sterile membrane 1 is adhered to the lens. The optional fixing means 3 of the membrane 1 have an adhesive similar to or different from that of the circular portion 2. The fixing means 3 are attached to a metal part of the camera in the region of the lens, which serves as a support for this lens, so as to reinforce the grip of the membrane 1 on the lens. The hydrophobic surface of the first face 4 of the membrane 1 prevents soiling originating from the surrounding biological fluids from adhering to this surface and hampering the operator's visibility. At the end of the intervention, the membrane 1 is manually removed from the lens, which will be sterilized for a new use.

Thus, the membrane 1 of the invention is simple to use, inexpensive and provides an efficient self-cleaning surface.

It goes without saying that the invention is not limited to the embodiments described above as examples, but comprises all the technical equivalents and variants of the described means and also combinations thereof.

## Claims

1. Disposable flexible membrane (1) suitable for adhesion to an objective lens, especially an objective of a camera for laparoscopic operations, the membrane (1) being transparent and comprising two opposite faces, a first face (4) comprising a hydrophobic surface, and a second face (6) comprising a surface of reversible adhesiveness, **characterized by** the hydrophobic surface comprising particles whose sizes range from 1 nanometre to 20 micrometres and whose surface is hydrophobic, and in that the particles comprise cellulose nanofibres.

2. Membrane (1) according to Claim 1, **characterized in that** the second face (6) comprises an adhesive material.

3. Membrane (1) according to Claim 2, **characterized in that** the adhesive material is chosen from epoxy adhesives and pressure-sensitive adhesive (PSA) polymers such as vinyl polymers, especially vinyl ethers, ethylene-vinyl acetates (EVA), natural and butyl rubbers, nitriles, acrylic resins, silicone rubbers, especially styrene-butadiene-styrene (SBS) copolymers, styrene block copolymers (SBC), styrene-ethylene/butylene-styrene (SEBS) copolymers, styrene-ethylene/propylene (SEP) copolymers and styrene-isoprene-styrene (SIS) copolymers, and mixtures thereof.

4. Membrane (1) according to any one of Claims 1 to 3, **characterized in that** the hydrophobic surface comprises a hydrophobic compound grafted onto the first face (4) or a hydrophobic compound in the form of a deposited layer (5).

5. Membrane (1) according to any one of Claims 1 to 4, **characterized in that** the hydrophobic surface comprises texturing, the dimensions of which range from 1 nanometre to 20 micrometres.

6. Membrane (1) according to any one of Claims 1 to 5, **characterized in that** the particles comprise the product of the reaction of cellulose nanofibres with a hydrophobic fluoro compound such as tridecafluoro(1,1,2,2-tetrahydrooctyl)-trichlorosilane.

7. Membrane (1) according to any one of Claims 1 to 6, **characterized in that** the membrane (1) comprises a flexible film (7) comprising a polymer material such as polyvinyl, poly(ethylene terephthalate), acrylic and mixtures thereof, a first surface (8) and an opposite second surface (9), the flexible film (7) being arranged between the first face (4) and the second face (6) of the membrane (1).

8. Membrane (1) according to Claim 7, **characterized in that** the flexible film (7) comprises a plasticizer.

9. Membrane (1) according to Claims 7 or 8, **characterized in that** the polymer material of the flexible film (7) is an adhesive polymer such as styrene-butadiene-styrene copolymer and forms the adhesive material of the second face (6) of the membrane (1).

10. Membrane (1) according to Claims 7 or 8, **characterized in that** the polymer material of the flexible film (7) is a hydrophobic material, such as a fluoro polymer, especially polytetrafluoroethylene, fluorinated ethylene-propylene, a fluorinated elastomer, and mixtures thereof, and forms the hydrophobic material of the first face (4) of the membrane (1).

11. Membrane (1) according to any one of Claims 1 to 10, **characterized in that** the membrane (1) also comprises fixing means adapted to be fixed onto a metal substrate serving as a support for the said lens.

12. Membrane (1) according to Claim 11, **characterized in that** the membrane (1) comprises a circular portion (2) intended to be adhered to the lens and **in that** the fixing means comprise at least one tab (3) extending radially to the circular portion (2).

13. Method for temporarily protecting the objective lens of a camera comprising the step of manually positioning a membrane according to any one of claims 1 to 12 onto said lens and further manually removing said membrane from said lens.

## Patentansprüche

1. Flexible Einwegmembran (1), die zum Verkleben an eine Objektivlinse, insbesondere ein Objektiv einer Kamera für laparoskopische Operationen, geeignet ist, wobei die Membran (1) transparent ist und zwei gegenüberliegende Flächen umfasst, wobei eine erste Fläche (4) eine hydrophobe Oberfläche und eine zweite Fläche (6) eine Oberfläche mit reversiblem Haftvermögen umfasst, **dadurch gekennzeichnet, dass** die hydrophobe Oberfläche Partikel umfasst, deren Größe im Bereich von 1 Nanometer bis 20 Mikrometer liegt und deren Oberfläche hydrophob ist, und dass die Partikel Cellulose-Nanofasern umfassen.

2. Membran (1) nach Anspruch 1, **dadurch gekennzeichnet, dass** die zweite Fläche (6) einen Klebstoff umfasst.

3. Membran (1) nach Anspruch 2, **dadurch gekennzeichnet, dass** das Klebematerial ausgewählt ist aus Epoxidklebstoffen und Haftklebstoff (PSA) -Polymeren wie Vinylpolymeren, insbesondere Vinylethern, Ethylen-Vinylacetat (EVA), natürlich und Butylkautschuke, Nitrile, Acrylharze, Siliconkautschuke, insbesondere Styrol-Butadien-Styrol (SBS) -Copolymere, Styrol-Blockcopolymere (SBC), Styrol-Ethylen / Butylen-Styrol (SEBS) -Copolymere, Styrol-Ethylen / Propylen (SEP) Copolymere und Styrol-Isopren-Styrol (SIS) -Copolymere und Gemische davon ausgewählt ist.

4. Membran (1) nach einem der Ansprüche 1 bis 3, **dadurch gekennzeichnet, dass** die hydrophobe Oberfläche eine hydrophobe Verbindung, die auf der ersten Seite (4) gepfropft ist, oder eine hydrophobe Verbindung in Form einer abgeschiedenen Schicht (5) umfasst.

5. Membran (1) nach einem der Ansprüche 1 bis 4, **dadurch gekennzeichnet, dass** die hydrophobe Oberfläche eine Texturierung aufweist, deren Abmessungen im Bereich 1 Nanometer bis 20 Mikrometer liegen.

6. Membran (1) nach einem der Ansprüche 1 bis 5, **dadurch gekennzeichnet, dass** die Partikel das Produkt der Umsetzung von Cellulose-Nanofasern mit einem hydrophoben Fluorverbindung wie Tridecafluor (1,1,2,2-tetrahydrooctyl)-Trichlorsilan umfassen.

7. Membran (1) nach einem der Ansprüche 1 bis 6, **dadurch gekennzeichnet, dass** die Membran (1) eine flexible Folie (7) umfasst, umfassend ein Polymermaterial, wie Polyvinylchlorid, Poly(ethylenterephthalat), Acryl und Gemische davon, eine erste Oberfläche (8) und eine gegenüberliegende zweite Oberfläche (9), wobei die flexible Folie (7) zwischen der ersten Seite (4) und der zweiten Seite (6) der Membran (1) angeordnet ist.

8. Membran (1) nach Anspruch 7, **dadurch gekennzeichnet, dass** die flexible Folie (7) einen Weichmacher umfasst.

9. Membran (1) nach den Ansprüchen 7 oder 8, **dadurch gekennzeichnet, dass** das Polymermaterial der flexiblen Folie (7) ein haftendes Polymer wie beispielsweise Styrol-Butadien-Styrol-Copolymer ist, und wobei das Haftmaterial der zweiten Fläche (6) der Membran (1) ausbildet.

10. Membran (1) nach den Ansprüchen 7 oder 8, **dadurch gekennzeichnet, dass** das Polymermaterial der flexiblen Folie (7) ein hydrophobes Material wie ein Fluorpolymer ist, insbesondere wie Polytetrafluorethylen, fluoriertes Ethylen-Propylen, ein fluoriertes Elastomer, und Gemische davon und dass das hydrophobe Material der ersten Seite (4) der Membran (1) ausbildet.

11. Membran (1) nach einem der Ansprüche 1 bis 10, **dadurch gekennzeichnet, dass** die Membran (1) ferner Befestigungsmittel umfasst, die geeignet sind, auf ein Metallsubstrat befestigt zu werden, das als Träger für die Linse dient.

12. Membran (1) nach Anspruch 11, **dadurch gekennzeichnet, dass** die Membran (1) einen kreisförmigen Abschnitt (2) umfasst, der zum Verkleben an der Linse vorgesehen ist, und dass das Befestigungsmittel mindestens eine Lasche (3) umfasst, die sich radial zu dem kreisförmigen Abschnitt (2) erstreckt.

13. Verfahren zum vorübergehenden Schützen der Objektivlinse einer Kamera, umfassend den Schritt des manuellen Positionierens einer Membran nach einem der Ansprüche 1 bis 12 auf die Linse, und ferner umfassend das manuelle Entfernen der Membran von der Linse.

## Revendications

1. Membrane flexible jetable (1) apte à adhérer à une lentille d'objectif, notamment l'objectif d'une caméra employée lors d'opérations laparoscopiques, la membrane (1) étant transparente et comportant deux faces opposées, une première face (4) comprenant une surface hydrophobe, et une seconde face (6) comprenant une surface d'adhésivité réversible, **caractérisée en ce que** la surface hydrophobe comprend des particules dont les tailles varient de 1 nanomètre à 20 micromètres et dont la surface est hydrophobe, et **en ce que** les particules comprennent des nanofibres de cellulose.

2. Membrane (1) selon la revendication 1, **caractérisée en ce que** la seconde face (6) comprend un matériau adhésif.

3. Membrane (1) selon la revendication 2, **caractérisée en ce que** le matériau adhésif est choisi parmi les adhésifs à base de résines époxydes et les polymères adhésifs autocollants (PSA) tels que les polymères vinyliques, notamment les éthers vinyliques, les acétates de vinyle-éthylène (EVA), les élastomères naturels et butyliques, les nitriles, les résines acryliques, les élastomères silicone, notamment les copolymères styrène-butadiène séquencés (SBS), les copolymères blocs styrène (SBC), les copolymères styrène-éthylène/butylène-styrène (SEBS), les copolymères styrène-éthylène/propylène (SEP) et les copolymères styrène-isoprène-styrène (SIS), et leurs mélanges.

4. Membrane (1) selon l'une quelconque des revendications 1 à 3, **caractérisée en ce que** la surface hydrophobe comprend un composé hydrophobe greffé sur la première face (4) ou un composé hydrophobe sous forme de couche déposée (5).

5. Membrane (1) selon l'une quelconque des revendications 1 à 4, **caractérisée en ce que** la surface hydrophobe comprend une texturation dont les dimensions varient de 1 nanomètre à 20 micromètres.

6. Membrane (1) selon l'une quelconque des revendications 1 à 5, **caractérisée en ce que** les particules comprennent le produit de la réaction de nanofibres de cellulose avec un composé fluoré hydrophobe tel que le tridécafluoro(1,1,2,2-tétrahydrooctyl)-trichlorosilane.

7. Membrane (1) selon l'une quelconque des revendications 1 à 6, **caractérisée en ce que** la membrane (1) comprend un film souple (7) comprenant un matériau polymère tel que le polyvinyle, le poly(éthylène téréphtalate), l'acrylique et leurs mélanges, une première surface (8) et une seconde surface opposée (9), le film souple (7) étant disposé entre la première face (4) et la seconde face (6) de la membrane (1).

8. Membrane (1) selon la revendication 7, **caractérisée en ce que** le film souple (7) comprend un plastifiant.

9. Membrane (1) selon les revendications 7 ou 8, **caractérisée en ce que** le matériau polymère du film souple (7) est un polymère adhésif tel que le copolymère styrène-butadiène séquencé et forme le matériau adhésif de la seconde face (6) de la membrane (1).

10. Membrane (1) selon les revendications 7 ou 8, **caractérisée en ce que** le matériau polymère du film souple (7) est un matériau hydrophobe, tel qu'un polymère fluoré, notamment du polytétrafluoréthylène, de l'éthylène-propylène fluoré, un élastomère fluoré, et leurs mélanges, et forme le matériau hydrophobe de la première face (4) de la membrane (1).

11. Membrane (1) selon l'une quelconque des revendications 1 à 10, **caractérisée en ce que** la membrane (1) comprend également des moyens de fixation aptes à être fixés sur un substrat métallique servant de support à ladite lentille.

12. Membrane (1) selon la revendication 11, **caractérisée en ce que** la membrane (1) comprend une partie circulaire (2) destinée à adhérer à la lentille et **en ce que** les moyens de fixation comprennent au moins une patte (3) s'étendant radialement jusqu'à la partie circulaire (2).

13. Procédé permettant de protéger temporairement la lentille d'objectif d'une caméra comprenant l'étape consistant à positionner manuellement une membrane selon l'une quelconque des revendications 1 à 12 sur ladite lentille et de retirer en outre manuellement ladite membrane de ladite lentille.
